(19) **Europäisches Patentamt / European Patent Office / Office européen des brevets**

(11) **EP 3 081 856 B1**

(12) # FASCICULE DE BREVET EUROPEEN

(45) Date de publication et mention de la délivrance du brevet:
**18.10.2017 Bulletin 2017/42**

(51) Int Cl.:
*F21V 21/40* *(2006.01)*  *F21V 23/04* *(2006.01)*
*F21L 14/04* *(2006.01)*  *H01H 36/00* *(2006.01)*

(21) Numéro de dépôt: **16165340.7**

(22) Date de dépôt: **14.04.2016**

(54) **DISPOSITIF D'ÉCLAIRAGE MÉDICAL**

MEDIZINISCHES BELEUCHTUNGSGERÄT

MEDICAL LIGHTING DEVICE

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **15.04.2015 FR 1553333**

(43) Date de publication de la demande:
**19.10.2016 Bulletin 2016/42**

(73) Titulaire: **Steris**
**33185 Le Haillan (FR)**

(72) Inventeur: **BESNARD, Mathieu**
**33160 SAINT MEDARD EN JALLES (FR)**

(74) Mandataire: **Robert, Mathias et al**
**Ernest Gutmann - Yves Plasseraud S.A.S.**
**3, rue Auber**
**75009 Paris (FR)**

(56) Documents cités:
**EP-A2- 1 342 947        WO-A2-2010/053882**
**DE-U1-202007 011 597    US-A1- 2006 109 650**
**US-A1- 2014 035 459     US-B1- 7 802 898**

- **Anonymous: "Rotary encoder - Wikipedia, the free encyclopedia", , 6 avril 2015 (2015-04-06), XP055293566, Extrait de l'Internet: URL:https://web.archive.org/web/2015040621 1339/http://en.wikipedia.org/wiki/Rotary_e ncoder [extrait le 2016-08-04]**

EP 3 081 856 B1

**EP 3 081 856 B1**

**Description**

La présente invention concerne un dispositif d'éclairage médical, notamment pour un bloc opératoire.

Un tel dispositif d'éclairage comporte classiquement un bras articulé dont une extrémité est fixe et dont une extrémité opposée mobile est équipée d'un module d'éclairage.

Le module d'éclairage peut être déplacé par un chirurgien, de manière à orienter le faisceau lumineux vers une zone à éclairer d'un patient.

Les besoins en quantité de lumière, en forme et en taille de la tâche lumineuse générée par le dispositif d'éclairage peuvent varier, notamment en fonction du type d'intervention pratiquée.

La demande de brevet EP 2 774 569 et la publication EP 1.342.947 divulguent un dispositif d'éclairage médical, notamment pour un bloc opératoire, comportant une tête ou coupole d'éclairage comprenant un corps dans lequel sont montées des sources lumineuses, une poignée montée sur le corps et mobile en rotation par rapport au corps autour de l'axe de la poignée.

La poignée est équipée d'un potentiomètre apte à émettre un signal indicatif de la position de la poignée, le signal étant envoyé à des moyens de commande aptes à commander un paramètre de réglage du dispositif, tel par exemple que l'intensité lumineuse.

L'utilisation d'un potentiomètre en tant que moyens de détection ne permet pas de garantir une grande fiabilité au dispositif.

L'invention a notamment pour but d'apporter une solution simple, efficace et économique à ce problème.

A cet effet, elle propose un dispositif d'éclairage médical, selon la revendication 1. L'utilisation de moyens de détection sans contact permet d'augmenter la fiabilité du dispositif. En effet, de tels moyens ne risquent pas d'être dégradés par frottement de pièces pivotant ou pouvant vibrer l'une par rapport à l'autre.

La course angulaire de la poignée peut être limitée par deux butées définissant deux positions extrêmes opposées de la poignée, les moyens de détection étant aptes à détecter respectivement une première et une seconde positions angulaires de détection de la poignée, décalées angulairement respectivement par rapport auxdites positions extrêmes.

De cette manière, on garantit que les première et seconde positions angulaires sont correctement détectées par les moyens de détection avant que la poignée arrive en butée lors de son pivotement.

Par ailleurs, le dispositif peut comporter des moyens d'indexage, aptes à exercer un couple résistant sur la poignée, dans au moins une position angulaire déterminée de la poignée, de façon à indexer ladite position déterminée.

Dans ce cas, les moyens d'indexage peuvent être aptes à indexer des positions situées entre les première et seconde positions angulaires de détection par exemple juste avant lesdites positions de détection, et/ou une position médiane de la poignée située entre lesdites positions angulaires de détection. L'indexation en position médiane et celles juste avant les positions de détection permettent de réduire le risque de contrôle intempestif de l'éclairage lors du positionnement de la tête d'éclairage dans l'espace par un opérateur.

De plus, la tête d'éclairage peut comporter au moins une source lumineuse centrale et au moins une source lumineuse périphérique, décalée latéralement par rapport à la source lumineuse centrale par rapport à la direction d'éclairage de la source lumineuse centrale, le dispositif comportant des moyens de commande aptes à allumer la source lumineuse centrale dans un premier mode d'éclairage et aptes à allumer la source lumineuse centrale et la source lumineuse périphérique dans au moins un deuxième mode d'éclairage, le passage du premier mode d'éclairage au deuxième mode d'éclairage étant effectué par pivotement de la poignée.

Dans ce cas, la source lumineuse centrale peut être apte à générer une première tâche lumineuse à une distance focale déterminée, la source lumineuse périphérique étant apte à générer une deuxième tâche lumineuse de plus grande dimension que la première tâche lumineuse et superposée, au moins en partie, à la première tâche lumineuse.

On définit par exemple par dimension de la tâche lumineuse, la dimension de la zone dont l'intensité lumineuse est supérieure à 10% de l'intensité maximale de la tâche. Cette dimension peut être le diamètre de la tâche si cette dernière présente une forme circulaire.

En outre, le dispositif peut comporter au moins un indicateur lumineux, tel par exemple qu'une diode électroluminescente, logée dans le corps et apte à fournir une information sur un mode de fonctionnement du dispositif, au moins une surface d'affichage visible depuis l'extérieur de la tête d'éclairage, au moins un guide lumineux comprenant une première extrémité située en regard et à distance de l'indicateur lumineux et une seconde extrémité située au niveau de la surface d'affichage, la lumière générée par ledit indicateur lumineux étant apte à être guidée jusqu'à la surface d'affichage par ledit guide lumineux.

Le dispositif peut également comporter des moyens de diffusion de la lumière issue de la source lumineuse, lesdits moyens de diffusion comportant au moins deux lentilles mobiles l'une par rapport à l'autre et comportant chacune une surface plane et une surface ondulée présentant des creux et des saillies, lesdites surfaces ondulées des lentilles étant agencées en regard l'une de l'autre, de façon à faire converger ou diverger les rayons lumineux qui les traversent en fonction de leurs positions respectives, le déplacement de l'une au moins des lentilles par rapport à l'autre étant

2

effectué par pivotement de la poignée.

L'invention sera mieux comprise et d'autres détails, caractéristiques et avantages de l'invention apparaîtront à la lecture de la description suivante faite à titre d'exemple non limitatif en référence aux dessins annexés dans lesquels :

- la figure 1 est une vue en perspective d'un dispositif d'éclairage selon une forme de réalisation de l'invention,
- la figure 2 est une vue éclatée, en perspective, d'une partie du dispositif de la figure 1,
- la figure 3 est une vue en perspective, de dessous, d'une partie du dispositif,
- les figures 4 et 5 sont des vues en perspective et en coupe de la tête d'éclairage du dispositif,
- la figure 6 est une vue éclatée, en perspective, d'un module d'éclairage,
- la figure 7 est une vue en perspective d'une partie d'un dispositif d'éclairage selon une deuxième forme de réalisation, équipé de moyens d'indexage de la poignée,
- la figure 8 est une vue du détail référencée A à la figure 7,
- la figure 9 est une vue en coupe d'une partie du dispositif des figures 7 et 8,
- la figure 10 est une vue en perspective d'une partie d'un dispositif selon une troisième forme de réalisation,
- la figure 11 est une vue en perspective d'une tête d'éclairage d'un dispositif selon une quatrième forme de réalisation,
- les figures 12 et 13 sont des vues éclatées, en perspective, du dispositif d'éclairage de la figure 11,
- les figures 14 et 15 illustrent le principe de diffusion de la tâche lumineuse pour deux lentilles mobiles l'une par rapport à l'autre, conformément à la quatrième forme de réalisation.

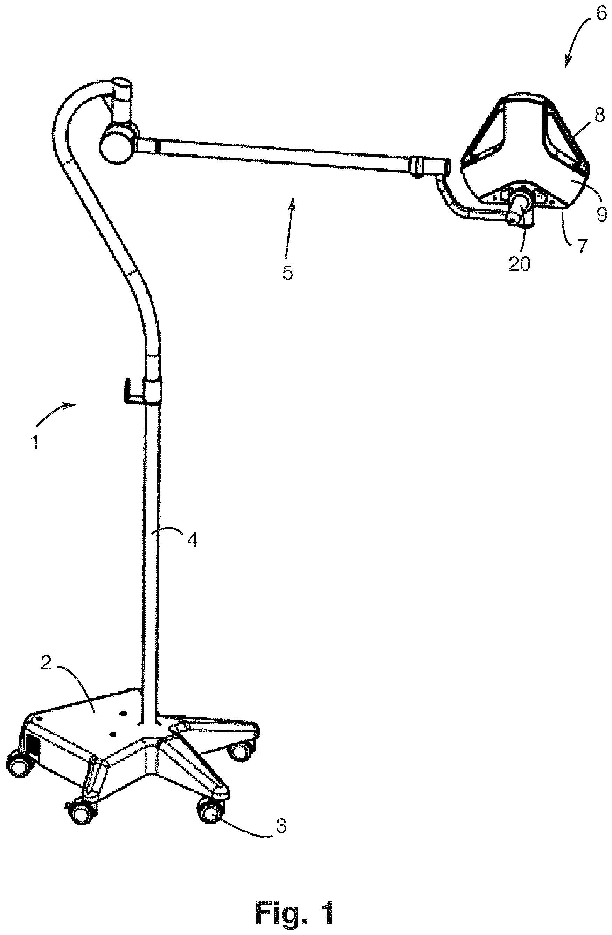

Les figures 1 à 6 représentent un dispositif d'éclairage médical 1, en particulier une lampe scialytique, notamment pour un bloc opératoire.

Le terme "bloc opératoire" inclut également les cas où le dispositif d'éclairage 1 est adapté à un fauteuil dentaire ou à un domaine d'activité similaire : salles d'examen, salles d'urgence, salles de plâtre, unités de soins intensifs, chambres de suture, chambres à induction, néonatologie, pédiatrie, maternité,.... Comme cela est visible à la figure 1, ce dispositif 1 comporte une embase mobile 2 équipée de roues 3, à partir de laquelle s'étend un mat 4, un bras 5 étant articulé sur l'extrémité supérieure du mat 4. Une tête ou coupole d'éclairage 6 est montée sur l'extrémité libre du bras 5. Le bras articulé 5 est formé de plusieurs éléments reliés les uns aux autres par des articulations permettant de déplacer manuellement la tête d'éclairage 6 en tout point de l'espace et d'orienter cette tête 6 dans toutes les directions.

Selon d'autres variantes non représentées, le bras articulé 5 est relié à un point fixe d'un mur ou d'un plafond.

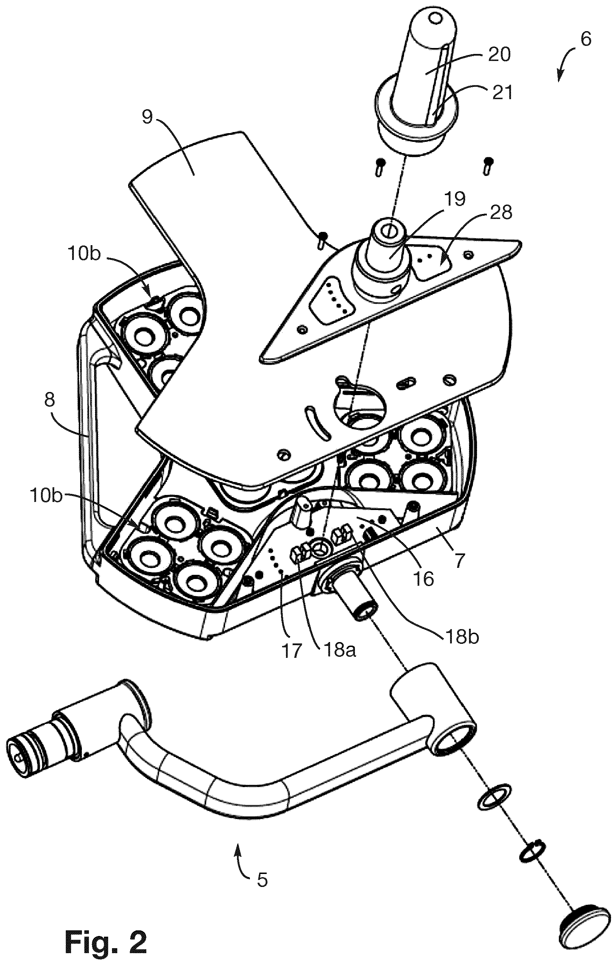

Comme cela est mieux visible aux figures 2 à 5, la tête d'éclairage 6 comporte un corps creux 7 de part et d'autre duquel sont montées des poignées fixes 8. Ces poignées 8 sont fixées à l'intérieur du corps 7 à l'aide de vis, de manière à pouvoir être démontées au besoin. L'ouverture du corps est recouverte d'un capot ou écran transparent de protection 9.

Des modules d'éclairage 10a, 10b sont montés à l'intérieur du corps 7. Plus particulièrement, la tête d'éclairage 6 comporte un module central 10a et quatre modules périphériques 10b, régulièrement répartis autour du module central 10a.

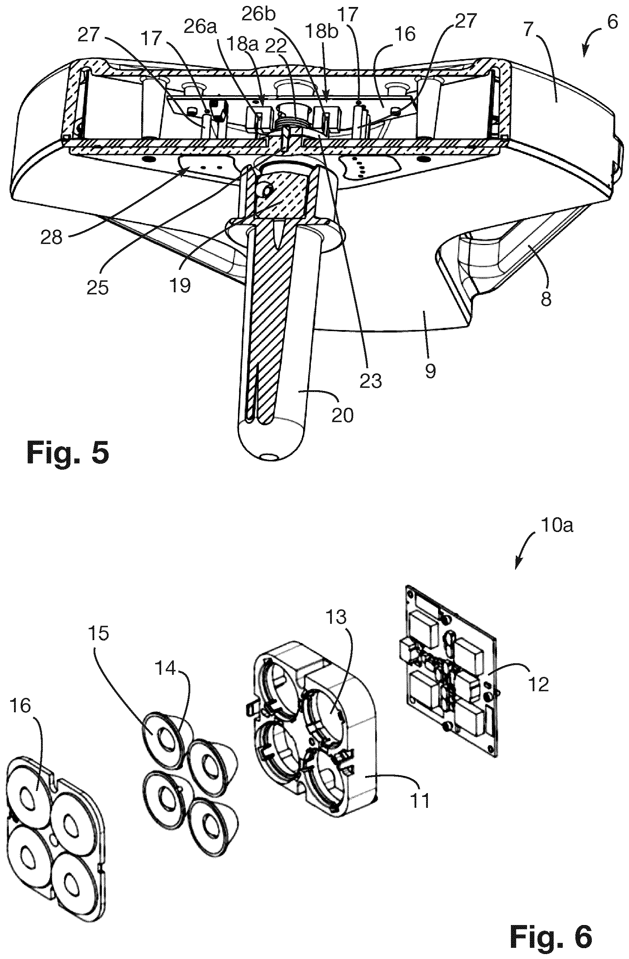

Comme illustré à la figure 6, chaque module 10a, 10b comporte un socle 11 monté sur une carte électronique 12, le socle 11 délimitant quatre logements 13 servant au montage de quatre sources lumineuses, à savoir des diodes électroluminescentes 14, équipées de collimateurs 15.

Les cartes électroniques 12 des modules 10a, 10b permettent de gérer l'allumage des différentes sources lumineuses.

Le module central 10a est équipé d'une plaque pourvue de de collimateurs additionnels 16 de façon à ce que la tâche de lumière formée par le module central 10a à une distance focale déterminée, ici de l'ordre de un mètre, présente une dimension ou un diamètre inférieur à la tâche de lumière formée par chacun des modules périphériques 10b. La tâche de lumière formée par le module central 10a a par exemple un diamètre de l'ordre de 16 cm alors que les tâches de lumière formées par chacun des modules périphériques 10b ont par exemple un diamètre de l'ordre de 22 cm, à la distance focale précitée. L'utilisation de plusieurs modules lumineux 10a, 10b décalés les uns des autres par rapport à l'axe du faisceau émis par le module central 10a permet d'obtenir un effet scialytique tendant à diluer ou réduire l'influence des ombres portées.

Le dispositif 1 comporte également des moyens de commande aptes notamment à commander l'allumage des différents modules 10a, 10b et comprenant une carte électronique de commande 16, montée et fixée dans le corps 7. Ladite carte électronique de commande 16 comporte des diodes électroluminescentes 17 (figures 2 et 5) aptes à fournir une indication quant au mode d'allumage ou de fonctionnement du dispositif 1.

Ladite carte électronique 16 comporte en outre une première et une seconde fourches optiques 18a, 18b, chaque fourche optique comportant un émetteur lumineux et un récepteur situé en regard de l'émetteur et apte à détecter un signal lumineux émis par ledit émetteur.

Un arbre 19a est monté de façon pivotante sur le corps 7. Un support de poignée 19b est solidaire de l'arbre 19a grâce à une vis 19c. Une poignée 20 est montée de façon amovible sur le support 19b. La poignée 20 est ainsi montée pivotante par rapport au corps 7 et peut être séparée de l'arbre 19a afin de pouvoir être changée ou stérilisée par exemple. La poignée 20 peut comporter une zone 21 en saillie longitudinale apte à faciliter la prise en main de la poignée 20 par un opérateur.

L'arbre 19a et la poignée 20 peuvent ainsi pivoter de part et d'autre d'une position angulaire médiane ou centrale. Des moyens de rappel élastiques 22 se présentant sous la forme d'un ressort de torsion tendent à ramener l'arbre 19a et la poignée 20 dans ladite position médiane.

Selon une autre forme de réalisation, le rappel de l'arbre 19a en position médiane peut être sans contact et magnétique avec au moins un aimant permanent monté sur l'arbre 19a, situé en regard d'une denture en matériau ferritique. L'usage de deux aimants permanents, à savoir un aimant mobile et solidaire de l'arbre 19a et un aimant fixe et solidaire du corps 7, agencés en regard l'un de l'autre et en attraction au niveau de la position médiane peut également être envisagé. Enfin l'usage de trois aimants permanents, à savoir un aimant mobile et solidaire de l'arbre 19a et deux aimants fixes en répulsion par rapport au premier, solidaires du corps 7 et placés à chaque extrémité du débattement de l'arbre 19a.

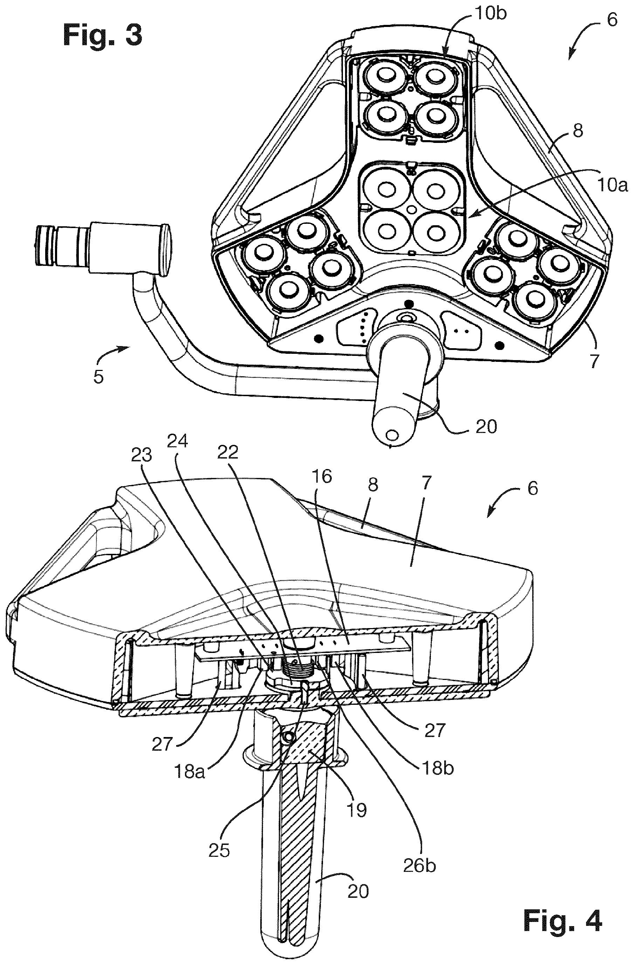

Comme cela est mieux visible à la figure 4, une rondelle 23 comportant une lumière ou une encoche circonférentielle 24 est fixée sur l'arbre 19a, un plot fixe 25 par rapport au corps 7 étant engagé dans la lumière ou l'encoche 24 de façon à pouvoir venir en butée sur les extrémités circonférentielles de la lumière ou de l'encoche 24.

La rotation de l'arbre 19a et de la poignée 20 est ainsi limitée par butée du plot 25 sur lesdites extrémités, entre une première et une seconde positions angulaires extrêmes de la poignée 20 par rapport au corps 7. Le débattement angulaire total de la poignée 20 par rapport au corps 7 est par exemple de l'ordre de 60°, soit 30° de débattement de part et d'autre de la position médiane.

Ladite rondelle 23 porte également une première et une seconde pattes 26a, 26b (figure 5) s'étendant parallèlement à l'axe de l'arbre 19a, en direction respectivement des fourches optiques 18a, 18b. Plus particulièrement, lors du pivotement de la poignée 20, la première patte 26a est apte à former un obstacle venant s'intercaler entre l'émetteur et le récepteur de la première fourche optique 18a, dans une première position angulaire de détection déterminée, la seconde patte 26b étant apte à former un obstacle venant s'intercaler entre l'émetteur et le récepteur de la seconde fourche optique 18b, dans une seconde position angulaire de détection déterminée. La première position angulaire de détection est située à proximité de la première position extrême, et légèrement décalée par rapport à cette position, par exemple d'un angle de 5°. De même, la seconde position de détection est située à proximité de la seconde position extrême, et légèrement décalée par rapport à cette position, par exemple d'un angle de 5°.

Le débattement angulaire entre les deux positions de détection est ainsi par exemple de l'ordre de 50°, soit 25° de part et d'autre de la position médiane.

L'utilisation de fourches optiques 18a, 18b et des pattes 26a, 26b permettent de fournir des indications quant à la position de la poignée 20, sans contact, de manière à augmenter la fiabilité du dispositif 1.

Des guides de lumineux tubulaires 27 (figure 5) sont montés dans le corps 7 et comprennent chacun une première extrémité située en regard et à distance de l'une des diodes électroluminescentes 17 de la carte électronique de commande 16 et une seconde extrémité débouchant au niveau d'une surface d'affichage 28 du corps 7, visible depuis l'extérieur. La lumière générée par chaque diode électroluminescente 17 peut ainsi être guidée jusqu'à la surface d'affichage 28 par le guide lumineux 27 correspondant. Ces guides 27 forment donc des moyens sans contact permettant d'amener une information lumineuse générée à l'intérieur du corps 7 jusque dans une zone 28 située à l'extérieur du corps 7. De tels moyens sans contact permettent ainsi d'augmenter la fiabilité du dispositif 1.

Le dispositif d'éclairage 1 est apte à fonctionner selon les modes suivants :

- un premier mode de fonctionnement dans lequel seul le module central 10a est allumé, de manière à former une tâche de type « spot » de faible diamètre,
- un second mode de fonctionnement dans lequel le module central 10a et l'un des modules périphériques 10b sont allumés simultanément de manière à former une tâche d'intensité et de diamètre plus importants que dans le premier mode,
- un troisième mode de fonctionnement dans lequel le module central 10a et deux des modules périphériques 10b sont allumés simultanément de manière à former une tâche de plus forte intensité que dans le deuxième mode,
- un quatrième mode de fonctionnement dans lequel le module central 10a et les trois modules périphériques 10b sont allumés simultanément de manière une tâche de plus forte intensité que dans le troisième mode,
- un cinquième mode de fonctionnement dans lequel tous les modules 10a, 10b sont éteints.

Le premier mode peut être activé par pivotement de la poignée 20 dans un premier sens de rotation, depuis la position centrale vers la première position, et par maintien de la première position pendant une durée déterminée, par exemple de l'ordre de 1 à 3 secondes.

La poignée 20 retrouve ensuite sa position médiane, par relâchement de la poignée 20, sous l'effet des moyens de rappel élastiques 22 ou magnétiques.

Les second, troisième et quatrième modes peuvent alors être activés successivement par pivotements successifs de la poignée 20 dans le premier sens de rotation, depuis la position centrale vers la première position, sans nécessiter de maintien de la première position pendant une durée déterminée.

Le cinquième mode peut être activé par pivotement de la poignée 20 dans un second sens de rotation de la poignée, opposé au premier sens de rotation, depuis la position centrale de la poignée vers la seconde position, et par maintien de la seconde position pendant une durée déterminée, par exemple de l'ordre de 1 à 3 secondes.

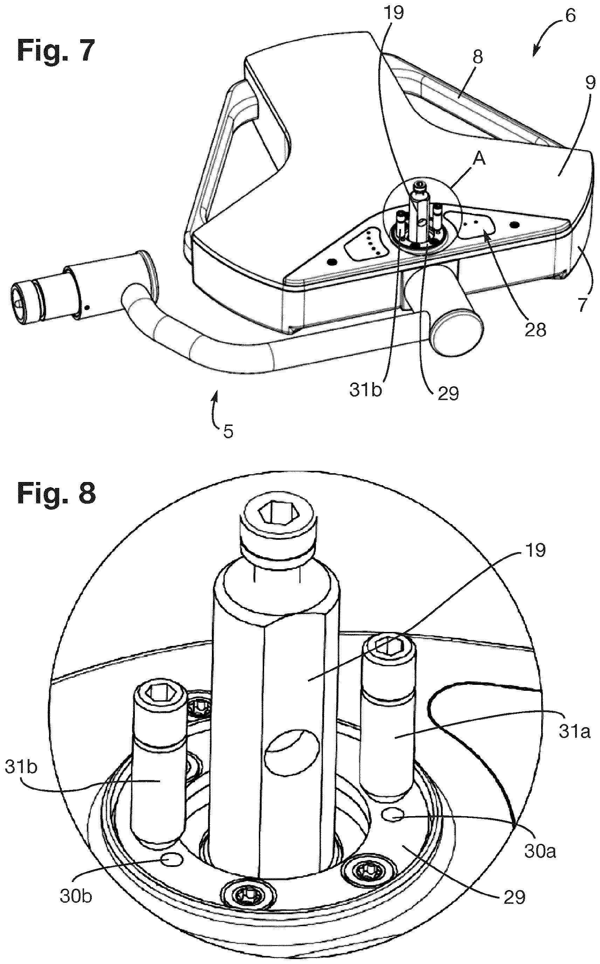

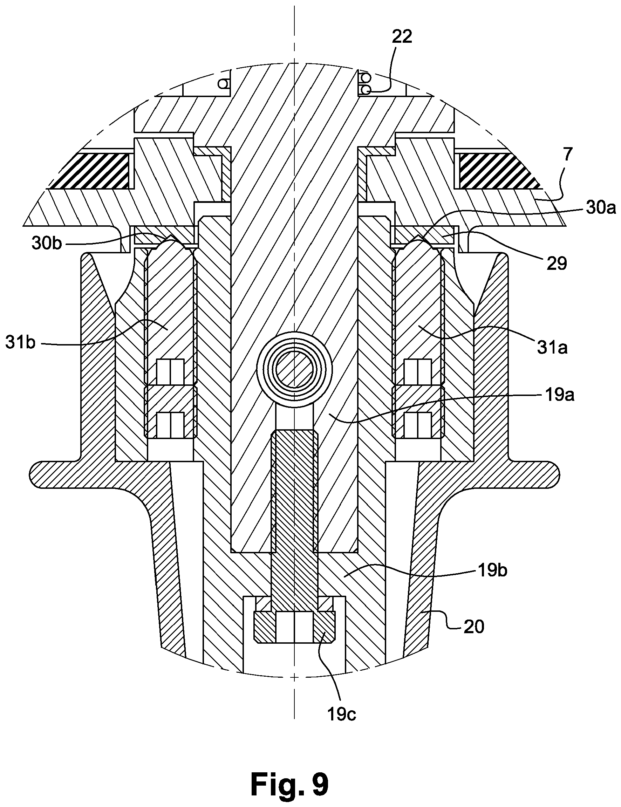

Afin d'améliorer l'ergonomie du dispositif 1, il est possible de prévoir des moyens d'indexage de la position de la poignée, comme cela est représenté aux figures 7 à 9.

Pour cela, une rondelle 29 équipée d'au moins un trou 30a peut être fixée sur le corps 7 et au moins un poussoir à ressort 31a peut être monté sur l'arbre 19a. Il est préférable de doubler le nombre de trous (30a et 30b) et le nombre de poussoirs à ressorts associés (31a et 31b) afin d'augmenter le couple résistant d'indexation et d'annuler le couple parasite sur l'arbre 19a. Chaque poussoir 31a, 31b comporte une bille soumise à l'action d'un organe élastique de rappel, la bille du premier poussoir 31a étant apte à être engagée dans le premier trou 30a, la bille du second poussoir 31b étant apte à être engagée dans le second trou 30b simultanément à l'engagement de la bille du premier poussoir 31a. Le but de l'indexation est de générer un couple résistant avant les positions de détection afin de réduire le risque de contrôle intempestif lors du positionnement de la tête d'éclairage dans l'espace par un opérateur.

Les premier et second poussoirs 31a, 31b sont ainsi aptes à exercer un couple résistant sur la poignée 30 dans au moins une position déterminée de la poignée 30, par exemple dans la position angulaire médiane de la poignée 30. De tels moyens d'indexage peuvent également être utilisés pour indexer d'autres positions, telles que par exemple juste avant la première et la seconde positions de détection.

Selon une autre forme de réalisation, l'indexation en position peut être sans contact et magnétique avec au moins un aimant permanent monté sur l'arbre 19a, situé en regard d'une denture en matériau ferritique. L'usage de deux aimants permanents, à savoir un aimant mobile et solidaire de l'arbre 19a et un aimant fixe et solidaire du corps 7, agencés en regard l'un de l'autre et en attraction au niveau de la position médiane peut également être envisagé. Enfin l'usage de trois aimants permanents, à savoir un aimant mobile et solidaire de 19a et deux aimants fixes en répulsion par rapport au premier, solidaires du corps 7 et placés à chaque extrémité du débattement de l'arbre 19a.

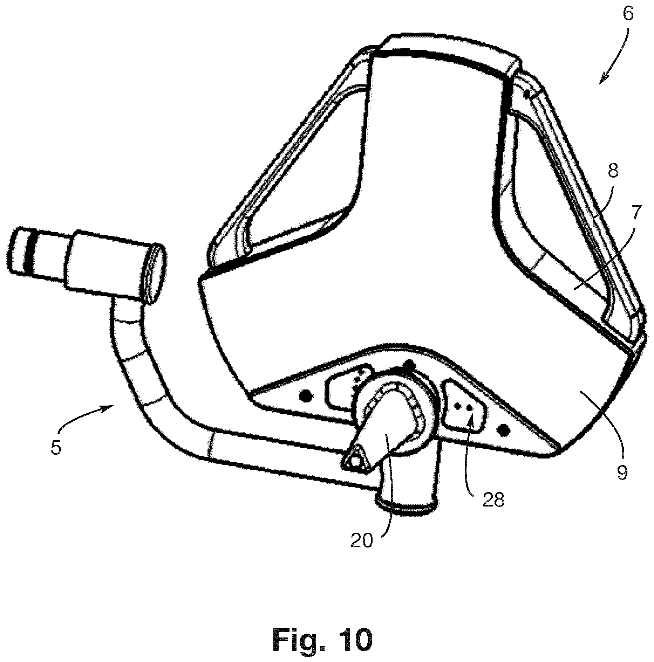

La figure 10 illustre un dispositif selon une autre forme de réalisation dans laquelle la poignée 20 présente une section polygonale, par exemple triangulaire, dans un plan perpendiculaire à l'axe de la poignée 20, de façon à faciliter la prise en main par un opérateur.

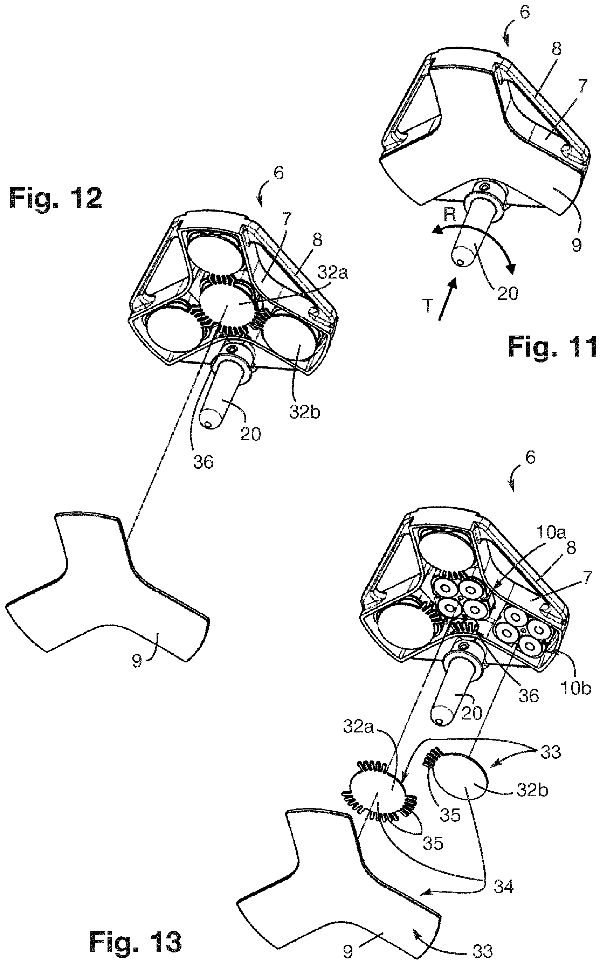

Les figures 11 à 13 illustrent un dispositif selon une autre forme de réalisation selon une autre de réalisation de l'invention, dans laquelle chaque module 10a, 10b est équipé d'une lentille mobile 32a, 32b par rapport à l'écran de protection 9.

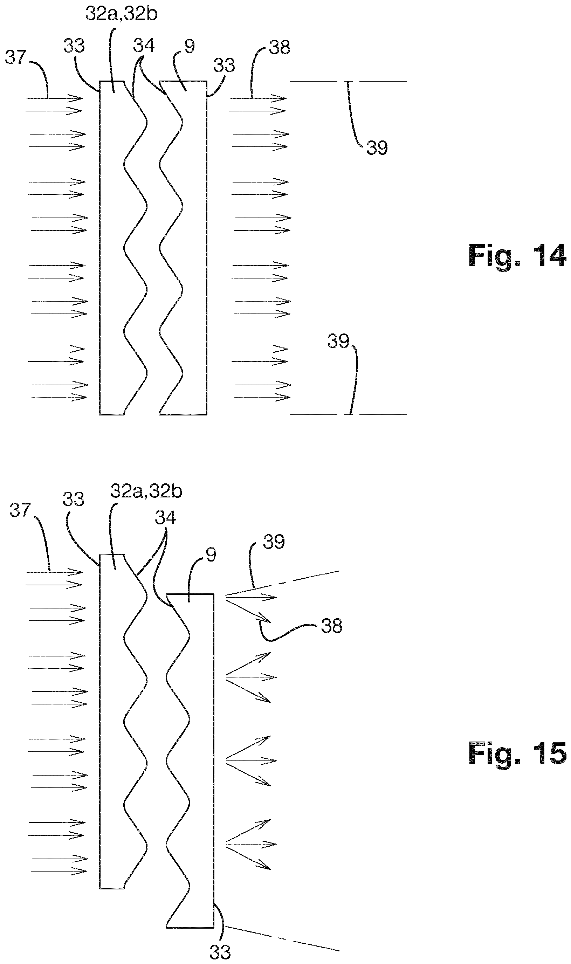

Les lentilles mobiles 32a, 32b comportent une surface plane 33 et une surface ondulée 34 présentant des creux et des saillies (figures 14, 15). Par ailleurs, l'écran de protection fixe 9 comporte une surface plane 33 et une surface ondulée 34.

Les surfaces ondulées 34 des lentilles 32a, 32b et de l'écran 9 sont agencées en regard l'une de l'autre, de façon à faire converger ou diverger les rayons lumineux qui les traversent en fonction de leurs positions respectives.

Les alternances de zones en creux et en saillie sont orientées dans deux directions, respectivement en direction circonférentielle et en direction radiale.

Les lentilles mobiles 32a, 32b se présentent sous la forme de disques présentant des moyens d'engrenage 35. La poignée 20 est également couplée à des moyens d'engrenage 36 aptes à engrener avec la lentille mobile 32a du module central 10a qui, elle-même, engrène avec toutes les lentilles mobiles 32b des modules périphériques 10b. Le pivotement de la poignée 20 permet donc, dans un mode de fonctionnement particulier décrit ci-après, de faire pivoter simultanément toutes les lentilles mobiles 32a, 32b par rapport aux modules fixes 10a, 10b ou par rapport à l'écran de protection 9 fixe.

Le principe général des moyens de diffusion à l'aide de deux plaques mobiles l'une par rapport à l'autre est connu du document US 5 775 799. L'invention permet d'adapter ce principe général au cas des modules précités.

Ce principe va maintenant être décrit en référence aux figures 14 et 15 représentent schématiquement une lentille mobile 32a, 32b et l'écran de protection fixe 9, dans deux positions différentes de la lentille mobile 32a, 32b.

En position de non diffusion, représentée à la figure 14, les lentilles 32a, 32b et l'écran 9 sont positionnés de telle manière que les creux de la lentille mobile 32a, 32b sont disposés en regard des saillies de l'écran fixe 9 et inversement. Les pas des creux et des saillies sont identiques d'une lentille à l'autre, aussi bien en direction circonférentielle qu'en direction radiale.

Dans cette position, les effets des deux lentilles s'annulent et le faisceau de lumière collimaté 37 provenant des diodes électroluminescentes émerge de l'écran 9 encore sensiblement collimaté (voir flèches 38), c'est-à-dire de manière non diffuse. Ceci est représenté par les lignes de référence 39 sur la figure 14.

Pour la diffusion de la lumière, les lentilles 32a, 32b et l'écran 9 sont positionnés de telle manière que les creux de la lentille mobile 32a, 32b sont disposés en regard des creux de l'écran fixe 9.

Dans cette position, les effets de divergence des deux lentilles se cumulent et le faisceau de lumière émerge de l'écran 9 de façon très diffuse. Ceci est représenté schématiquement par les lignes de référence 39 sur la figure 15.

Des positions intermédiaires de la lentille mobile 32a, 32b vis-à-vis de l'écran 9 permettent d'obtenir une diffusion plus moins ou moins importante du flux de lumière, et donc une tâche lumineuse plus ou moins grande.

La surface ondulée de la chaque lentille est une nappe sinusoïdale qui peut être exprimée en coordonnées sphériques sous la forme mathématique suivante :

$$R * \left[ 1 + K * \cos\left(\varphi_{\min} + D_\varphi * \varphi\right) * \cos\left( P_\varphi * \varphi * \frac{\cos(\varphi_{\min})}{\cos\left(\varphi_{\min} + \dfrac{D_\varphi * \varphi}{2}\right)} \right) * \cos(P_\theta * \theta) \right]$$

dans laquelle :

$\theta$ est l'angle de longitude,

$\varphi$ est l'angle de latitude,
R est le rayon de courbure (galbe) des lentilles,
K est l'amplitude relative de la nappe sinusoïdale,
$P\theta$ est la période selon l'angle $\theta$,
$P\varphi$ est la période selon l'angle $\varphi$,
$D\varphi$ est la différence de latitude entre les bords longitudinaux de la surface ondulée,

$\varphi$min est l'angle de latitude minimal.

En ajustant la formule mathématique du paragraphe précédent aux paramètres dimensionnels du module d'éclairage, on obtient la surface optique ondulée recherchée, qui est ensuite appliquée sur les surfaces 34 en regard des lentilles 32a, 32b et de l'écran 9.

La période des ondulations dépend préférentiellement du diamètre apparent des sources lumineuses, c'est-à-dire des diodes électroluminescentes 14. Il est en effet préférable qu'une demi-période d'ondulation (bosse ou creux) soit entièrement contenue dans le diamètre apparent d'une source lumineuse.

Préférentiellement, le diamètre apparent de la source lumineuse est beaucoup plus grand qu'une demi-période de la surface ondulée 34. Cette période influe également sur l'amplitude de déplacement, entre les lentilles 32a, 32b et l'écran 9 nécessaire pour le passage de la position de non-diffusion (figure 14) à la position de diffusion (figure 15).

On notera que l'amplitude des ondulations est sensiblement constante, quel que soit l'angle de longitude. Afin d'augmenter le diamètre de la tâche de lumière, il est notamment possible d'augmenter l'amplitude des ondulations. Si l'on cherche à obtenir une trop grande tâche de lumière, c'est-à-dire une trop grande diffusion du flux lumineux émis par les diodes électroluminescentes 14, on observe que la tâche comporte en son centre une zone dont l'intensité lumineuse est plus faible, appelée zone « d'ombre ».

Afin d'éviter un tel phénomène, une variante de réalisation consiste à faire varier l'amplitude des ondulations en fonction de la longitude de sorte que la surface ondulée 34 présente une zone très diffusante, permettant d'obtenir une tâche large avec une zone d'ombre centrale, et une zone moins diffusante, permettant d'obtenir une tâche de diamètre plus faible éclairant la zone d'ombre. D'un point de vue mathématique, cette opération consiste à remplacer l'amplitude relative K dans la formule précitée par une fonction K($\theta$) qui dépend de la longitude $\theta$ de manière non linéaire.

On définit par exemple par diamètre de la tâche lumineuse, le diamètre de la zone dont l'intensité lumineuse est supérieure à 10% de l'intensité maximale.

On pourrait élargir encore davantage la tâche lumineuse, mais cela n'est pas utile pour les applications médicales usuelles et l'usinage des ondulations serait dans ce cas plus complexe à réaliser.

Dans cette forme de réalisation, la poignée 20 est mobile en translation le long de son axe, comme illustré par la flèche T à la figure 11, entre deux positions axiales. Une première position permet de sélectionner l'un des cinq modes de fonctionnement décrit précédemment, une seconde position permettant de faire varier la position des lentilles mobiles 32a, 32b par rapport aux lentilles fixes 15, 16, par pivotement de la poignée 20 (illustré par la flèche R à la figure 11).

Le pivotement de la poignée 20, et donc le déplacement des lentilles mobiles 32a, 32b par rapport à l'écran fixe 9, sont limités par la présence des butées 24, 25.

**Revendications**

1. Dispositif d'éclairage médical (1), notamment pour un bloc opératoire, comportant une tête d'éclairage (6) comprenant un corps (7) dans lequel est montée au moins une source lumineuse (14) apte à générer un faisceau lumineux, une poignée (20) s'étendant selon un axe, montée sur le corps (7) et mobile en rotation par rapport au corps (7) autour de l'axe de ladite poignée (20), des moyens de commande (16, 12) situés dans le corps (7) et aptes à commander un paramètre d'éclairage, des moyens de détection (18a, 18b, 26a, 26b) de la position angulaire de la poignée (20), les moyens de détection comportant au moins une première partie (26a, 26b) couplée en rotation par rapport à la poignée (20) et au moins une seconde partie (18a, 18b) couplée en rotation par rapport au corps (7) et apte à fournir aux moyens de commande (16, 12) un signal qui est représentatif de la position angulaire la poignée (20) par rapport au corps (7), la seconde partie (18a, 18b) étant apte à détecter la position angulaire de la première partie (26a, 26b) sans contact, ou inversement, et les moyens de détection comportant au moins des première et seconde fourches optiques (18a, 18b) aptes à détecter deux positions angulaires différentes de la poignée (20), chaque fourche optique (18a, 18b) comprenant un émetteur lumineux et un récepteur situé en regard de l'émetteur et apte à détecter un signal lumineux émis par ledit émetteur, les moyens de détection comportant en outre des première et seconde pattes formant obstacles (26a, 26b), la première patte (26a) étant apte à venir s'intercaler, entre l'émetteur et le récepteur de la première fourche optique (18a) dans une première position angulaire de la poignée (20), la seconde patte (26b) étant apte à venir s'intercaler entre l'émetteur et le récepteur de la seconde fourche optique (18b) dans une seconde position angulaire de la poignée (20).

2. Dispositif (1) selon la revendication 1, **caractérisé en ce que** la course angulaire de la poignée (20) est limitée par deux butées (24, 25) définissant deux positions extrêmes opposées de la poignée (20), les moyens de détection (18a, 18b, 26a, 26b) étant aptes à détecter respectivement une première et une seconde positions angulaires de détection de la poignée (20), décalées angulairement respectivement par rapport auxdites positions extrêmes.

3. Dispositif (1) selon la revendication 1 ou 2, **caractérisé en ce qu'**il comporte des moyens d'indexage (30a, 30b, 31a, 31b), aptes à exercer un couple résistant sur la poignée (20), dans au moins une position angulaire déterminée de la poignée (20), de façon à indexer ladite position déterminée.

4. Dispositif (1) selon les revendications 2 et 3, **caractérisé en ce que** les moyens d'indexage sont aptes à indexer des positions situées entre les première et seconde positions angulaires de détection.

5. Dispositif (1) selon l'une des revendications 1 à 4, **caractérisé en ce que** la tête d'éclairage (6) comporte au moins une source lumineuse centrale (14, 10a) et au moins une source lumineuse périphérique (14, 10b), décalée latéralement par rapport à la source lumineuse centrale (14, 10a) par rapport à la direction d'éclairage de la source lumineuse centrale 14, 10a), le dispositif (1) comportant des moyens de commande (16, 12) aptes à allumer la source lumineuse centrale (14, 10a) dans un premier mode d'éclairage et aptes à allumer la source lumineuse centrale (14, 10a) et la source lumineuse périphérique (14, 10b) dans au moins un deuxième mode d'éclairage, le passage du premier mode d'éclairage au deuxième mode d'éclairage étant effectué par pivotement de la poignée (20).

6. Dispositif (1) selon la revendication 5, **caractérisé en ce que** la source lumineuse centrale (14, 10a) est apte à générer une première tâche lumineuse à une distance focale déterminée, la source lumineuse périphérique (14, 10b) étant apte à générer une deuxième tâche lumineuse de plus grande dimension que la première tâche lumineuse et superposée, au moins en partie, à la première tâche lumineuse.

7. Dispositif (1) selon l'une des revendications 1 à 6, **caractérisé en ce qu'**il comporte au moins un indicateur lumineux (17), tel par exemple qu'une diode électroluminescente (17), logée dans le corps (10) et apte à fournir une information sur un mode de fonctionnement du dispositif (1), au moins une surface d'affichage (28) visible depuis l'extérieur de la tête d'éclairage (6), au moins un guide lumineux (27) comprenant une première extrémité située en regard et à distance de l'indicateur lumineux (17) et une seconde extrémité située au niveau de la surface d'affichage (28), la lumière générée par ledit indicateur lumineux (17) étant apte à être guidée jusqu'à la surface d'affichage (28) par ledit guide lumineux (27).

8. Dispositif (1) selon l'une des revendications 1 à 7, **caractérisé en ce qu'**il comporte des moyens de diffusion de la lumière issue de la source lumineuse (14, 10a, 10b), lesdits moyens de diffusion comportant au moins deux lentilles (32a, 32b ; 9) mobiles l'une par rapport à l'autre et comportant chacune une surface plane (33) et une surface ondulée (34) présentant des creux et des saillies, lesdites surfaces ondulées (34) des lentilles (32a, 32b ; 9) étant agencées en regard l'une de l'autre, de façon à faire converger ou diverger les rayons lumineux qui les traversent en fonction de leurs positions respectives, le déplacement de l'une au moins des lentilles par rapport à l'autre étant effectué par pivotement de la poignée (20).

**Patentansprüche**

1. Medizinische Beleuchtungsvorrichtung (1), insbesondere für einen OP-Saal, welche einen Beleuchtungskopf (6) mit einem Körper (7) umfasst, in den mindestens eine Lichtquelle (14) montiert ist, welche einen Lichtstrahl erzeugen kann, ein Griff (20), der sich an einer Achse entlang erstreckt, auf den Körper (7) montiert ist und in Bezug zum Körper (7) um die Achse besagten Griffs (20) drehbar ist, am Körper (7) befindliche Steuerungsmittel (16, 12), die einen Beleuchtungsparameter steuern können, Detektionsmittel (18a, 18b, 26a, 26b) zur Erkennung der Winkelposition des Griffs (20), wobei die Detektionsmittel mindestens einen ersten Teil (26a, 26b) umfassen, der relativ zum Griff (20) drehbar gelagert ist, und mindestens einen zweiten Teil (18a, 18b), der relativ zum Körper (7) drehbar gelagert ist und den Steuerungsmitteln (16, 12) ein Signal senden kann, das repräsentativ für die Winkelposition des Griffs (20) relativ zum Körper (7) ist, wobei der zweite Teil (18a, 18b) in der Lage ist, die Winkelposition des ersten Teils (26a, 26b) kontaktlos zu erkennen, oder umgekehrt, die Detektionsmittel, welche mindestens eine erste und eine zweite Gabellichtschranken (18a, 18b) enthalten, in der Lage sind, zwei unterschiedliche Winkelpositionen des Griffs (20) zu erkennen, wobei jede optische Gabellichtschranke (18a, 18b) einen Lichtsender und einen Empfänger gegenüber dem Sender aufweist, der in der Lage ist, ein von besagtem Sender ausgesendetes Lichtsignal zu empfangen, wobei die Detektionsmittel des Weiteren einen ersten und einen zweiten blockierenden Bügel (26a, 26b) aufweisen, wobei der erste Bügel (26a) in der Lage ist, sich zwischen Sender und Empfänger der ersten optischen Gabellichtschranke (18a) in eine erste Winkelposition des Griffs (20) zu stellen, und der zweite Bügel (26b) in der Lage ist, sich zwischen den Sender und den Empfänger der zweiten Gabellichtschranke (18b) in eine zweite Winkelposition des Griffs (20) zu stellen.

2. Vorrichtung (1) nach Anspruch 1, **dadurch gekennzeichnet, dass** der Schwenkwinkel des Griffs (20) durch zwei Anschläge (24, 25) begrenzt wird, welche zwei einander gegenüberliegende Endstellungen des Griffs (20) abgrenzen, wobei die Detektionsmittel (18a, 18b, 26a, 26b) in der Lage sind, jeweils eine erste und eine zweite Winkeldetektionsposition des Griffs (20), die jeweils im Verhältnis zu den besagten Endpositionen winkelförmig versetzt sind, zu erkennen.

3. Vorrichtung (1) nach Anspruch 1 oder 2, **dadurch gekennzeichnet, dass** sie Arretierungsmittel (30a, 30b, 31a, 31b) umfasst, die in der Lage sind, in mindestens einer bestimmten Winkelposition des Griffs (20) ein Gegenmoment am Griff (20) zu erzeugen, so dass besagte bestimmte Position arretiert wird.

4. Vorrichtung (1) nach Anspruch 2 oder 3, **dadurch gekennzeichnet, dass** die Arretierungsmittel in der Lage sind, Positionen zwischen der ersten und der zweiten Winkeldetektionsposition zu arretieren.

5. Vorrichtung (1) nach einem der Ansprüche 1 bis 4, **dadurch gekennzeichnet, dass** der Beleuchtungskopf (6) mindestens eine mittlere Lichtquelle (14, 10a) und mindestens eine Lichtquelle am Rand (14, 10b) aufweist, die im Verhältnis zur mittleren Lichtquelle (14, 10a) relativ zur Beleuchtungsrichtung der mittleren Lichtquelle (14, 10a) seitlich verschoben ist, wobei die Vorrichtung (1) Steuerungsmittel (16, 12) umfasst, die die mittlere Lichtquelle (14, 10a) in einer ersten Beleuchtungsart und die mittlere Lichtquelle (14, 10a) und die Lichtquelle am Rand (14, 10b) in mindestens einer zweiten Beleuchtungsart einschalten können, wobei der Wechsel von der ersten Beleuchtungsart zur zweiten Beleuchtungsart durch Drehen des Griffs (20) erfolgt.

6. Vorrichtung (1) nach Anspruch 5, **dadurch gekennzeichnet, dass** die mittlere Lichtquelle (14, 10a) einen ersten Lichtfleck in einer bestimmten Brennweite erzeugen kann, wobei die Lichtquelle am Rand (14, 10b) einen zweiten größeren Lichtfleck als den ersten erzeugen kann, der den ersten Lichtfleck zumindest teilweise überlagert.

7. Vorrichtung (1) nach einem der Ansprüche 1 bis 6, **dadurch gekennzeichnet, dass** sie mindestens eine Leuchtanzeige (17), wie z.B. eine Leuchtelektrode (17), aufweist, die im Körper (10) aufgenommen ist und eine Information über eine Betriebsart der Vorrichtung (1) liefern kann, mindestens eine Anzeigefläche (28), die von der Außenseite des Beleuchtungskopfes (6) sichtbar ist, mindestens einen Lichtleiter (27), dessen erstes Ende sich gegenüber und in einem Abstand von der Leuchtanzeige (17) und dessen zweites Ende sich an der Anzeigefläche (28) befindet, wobei das von der Leuchtanzeige (17) generierte Licht vom Lichtleiter (27) bis zur Anzeigefläche (28) geführt werden kann.

8. Vorrichtung (1) nach einem der Ansprüche 1 bis 7, **dadurch gekennzeichnet, dass** sie Mittel zur Streuung des Lichts der Lichtquellen (14, 10a, 10b) umfasst, wobei besagte Streuungsmittel mindestens zwei gegeneinander bewegliche Linsen (32a, 32b; 9) enthalten, die jeweils eine ebene Fläche (33) und eine gewellte Fläche (34) mit Wellenbergen und -tälern aufweisen, wobei die besagten gewellten Flächen (34) der Linsen (32a, 32b; 9) gegenüberstehend angeordnet sind, so dass die Lichtstrahlen, die sie je nach Position durchqueren, konvergieren oder divergieren, wobei mindestens eine der Linsen im Verhältnis zur anderen durch Drehen des Griffes (20) verschoben wird.

**Claims**

1. A medical lighting device (1), especially for an operating theater, including a lighting head (6), comprising a body (7), wherein at least one light source (14) is mounted, which is adapted to generate a light beam, with a handle (20) extending along an axis, mounted on the body (7) and rotatable relative to the body (7) about the axis of said handle (20), control means (16, 12) located in the body (7) and adapted to control a lighting parameter, means (18a, 18b, 26a, 26b) for detecting the angular position of the handle (20), with the detection means including at least one first portion (26a, 26b) rotatably coupled relative to the handle and at least one second portion (18a, 18b) rotatably coupled relative to the body (7) and which is adapted to supply the control means (16, 12) with a signal which is representative of the angular position of the handle (20) relative to the body (7), with the second portion (18a, 18b) being adapted to detect the angular position of the first portion (26a, 26b) with no contact, or vice versa, and the detection means including at least one first and one second optical forks (18a, 18b) adapted to detect two different angular positions of the handle (20), with each optical fork (18a, 18b) comprising a light emitter and a receiver positioned opposite the emitter and which is adapted to detect a light signal emitted by said emitter, with the detection means further including first and second lugs which form obstacles (26a, 26b), with the first lug (26a) being adapted to be inserted between the emitter and the receiver of the first optical fork (18a) in a first angular position of the handle (20), with the second lug (26b) being adapted to be inserted between the emitter and the receiver of the second optical fork (18b) in a second angular position of the handle (20).

2. A device (1) according to claim 1, **characterized in that** the angular travel of the handle (20) is limited by two stops (24, 25) defining two opposite extreme positions of the handle (20), with the detection means (18a, 18b, 26a, 26b) being adapted to respectively detect a first and a second angular detection positions of the handle (20), angularly offset respectively relative to said extreme positions.

3. A device (1) according to claim 1 or 2, **characterized in that** it includes indexing means (30a, 30b, 31a, 31b), which is adapted to exert a resisting torque onto the handle (20), in at least one determined angular position of the handle (20), so as to index said determined position.

4. A device (1) according to claims 2 and 3, **characterized in that** the indexing means is adapted to index positions between the first and second angular detection positions.

5. A device (1) according to one of claims 1 to 4, **characterized in that** the lighting head (6) includes at least one central light source (14, 10a) and at least one peripheral light source (14, 10b), laterally offset relative to the central light source (14, 10a) relative to the lighting direction of the central light source (14, 10a), with the device (1) including control means (16, 12) which is adapted to turn on the central light source (14, 10a) in a first lighting mode and adapted to turn on the central light source (14, 10a) and the peripheral light source (14, 10b) in at least a second lighting mode, with the switching from the first lighting mode to the second lighting mode being executed by pivoting the handle (20).

6. A device (1) according to claim 5, **characterized in that** the central light source (14, 10a) is adapted to generate a first light spot at a determined focal length, with the peripheral light source (14, 10b) being adapted to generate a second light spot larger in size than the first light spot at least partially superimposed to the first light spot.

7. A device (1) according to one of claims 1 to 6, **characterized in that** it includes at least one light indicator (17), such as for example a light emitting diode (17), accommodated in the body (10) and adapted to provide information on an operating mode of the device (1), at least one display surface (28) visible from outside the lighting head (6), at least one light guide (27) comprising a first end located opposite and at a distance from the light indicator (17) and a second end located at the display surface (28), with the light generated by said light indicator (17) being adapted to be guided up to the display surface (28) by said light guide (27).

8. A device (1) according to one of claims 1 to 7, **characterized in that** it comprises means for diffusing the light from the light source (14, 10a, 10b), with said diffusing means having at least two lenses (32a, 32b; 9) movable with respect to one another and each including a flat surface (33) and a corrugated surface (34) having recesses and protrusions, with said corrugated surfaces (34) of the lenses (32a, 32b; 9) being arranged opposite one another, so as to make the light rays passing therethrough converge or diverge, according to the respective positions thereof, with the movement of the at least one lens relative to the other being executed by pivoting the handle (20).

**Fig. 1**

**Fig. 2**

**Fig. 3**

10b

6

8

10a

5

7

20

23 24 22 16 8 7 6

27

27

18a

18b

25 19 26b

20

**Fig. 4**

**Fig. 5**

**Fig. 6**

**Fig. 7**

**Fig. 8**

22

7

30a

30b

29

31b

31a

19a

19b

20

19c

**Fig. 9**

Fig. 10

**Fig. 12**

**Fig. 11**

**Fig. 13**

Fig. 14

Fig. 15

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 2774569 A
- EP 1342947 A
- US 5775799 A